# EUROPEAN PATENT SPECIFICATION

(11) **EP 2 623 053 B1**
(45) Date of publication and mention of the grant of the patent: **07.10.2015**
(21) Application number: 11828033.8
(22) Date of filing: 24.08.2011
(51) Int. Cl.: A61B 17/3209, A61B 17/16

(54) **SURGICAL MILLING CUTTER BRACKET**
KLAMMER FÜR EIN CHIRURGISCHES FRÄS- UND SCHNEIDEWERKZEUG
SUPPORT DE FRAISE CHIRURGICALE

(30) Priority: 30.09.2010 CN 201010298081
(43) Date of publication of application: 07.08.2013
(73) Proprietor: CHONGQING RUNZE PHARMACEUTICAL CO., LTD., Yubei District Chongqing 401120 (CN)
(72) Inventor: YE, Lei, Chongqing 401120 (CN); ZHOU, Jian, Chongqing 401120 (CN); FENG, Hua, Chongqing 401120 (CN); LI, Fei, Chongqing 401120 (CN); ZHU, Hengyang, Chongqing 401120 (CN); LI, Congxiao, Chongqing 401120 (CN)
(74) Representative: Style, Kelda Camilla Karen
(86) International application number: PCT/CN2011/078850
(87) International publication number: WO 2012/041137

(56) References cited:
- EP-A1- 0 714 634
- CN-A- 101 194 850
- CN-A- 101 194 850
- CN-A- 101 926 672
- CN-A- 101 940 490
- CN-A- 102 059 378
- CN-U- 201 798 785
- CN-U- 201 814 633
- CN-U- 201 861 715
- CN-Y- 2 862 991
- CN-Y- 2 862 991
- US-B2- 6 916 322

## Description

### Field of the Invention

The invention relates to a surgical cutting instrument and, more particularly, to a surgical milling cutter bracket.

### Description of the Prior art

During an operation, a milling cutter is used to perform some work of cutting. The milling cutter includes a milling cutter bracket, a cutting edge and an electric motor, etc. An L-shaped supporting bracket having a hole provided on the short side is provided on the top of the milling cutter bracket, a cylindrical bit on the front of the milling cutter bit projects into the hole, which ensures a preferred force bearing effect for the milling cutter. To protect the milling cutter, the cylindrical bit partially projects into the hole with some of the cylindrical bit left at the outside of the hole. In prior art, the short side of the L-shaped supporting bracket is designed to be a plane structure. When cutting an object, the cylindrical bit would not function if it contacts the object, which would cause uneven cutting to the object. Additionally, the structure :akes it difficult for the milling cutter to turn direction during the cutting process, which brings inconvenience to using the milling cutter.

Document CN 101194850 A discloses a hand milling machine comprising a milling cutter bracket.

### Summary of the Invention

The object of the invention is to provide a surgical milling cutter bracket which has the cylindrical bit not contacting the object to be cut and which also can turn direction flexibly

To achieve the object, the following technical scheme is adopted.

A surgical milling cutter bracket includes a retaining base with a through hole, wherein
a finger guide with an L-shaped supporting bracket at its top is disposed at the upper part of the retaining base, a protrusion is extending downwardly from the end of the short side of the L-shaped supporting bracket, and the lowest point of the protrusion is lower than the lowest point of a cylindrical bit of the milling cutter during the normal operation of the milling cutter;
a bolt with a through hole in its center is fixed in the cavity of the finger guide; a pressing plate is disposed at the upper part of the retaining base, and the bolt passes through the hole of the pressing plate to connect to a nut.

A finger guide is disposed at the lower part of the retaining base, a T-shaped bolt is fixed in the cavity of the finger guide, and a pressing plate is disposed at the upper part of the retaining base, and the nut passes through the hole of the pressing plate to connect to the bolt.

To ensure the smooth motion of the milling cutter, a bearing is disposed at the upper part of the finger guide.

To avoid the excessively flexibility of the finger guide, a spring sleeves the bolt.

To prevent skidding, the outer surfaces of the retaining base and finger guide are both provided with anti-slip grooves.

The invention adopts the protrusion to prevent the object from contacting the cylindrical bit, thus to avoid cutting the object unevenly. Meanwhile, the finger guide may drive the L-shaped bracket to rotate to change the motion direction of the milling cutter, which facilitates the operation.

### Brief Description of the Drawings

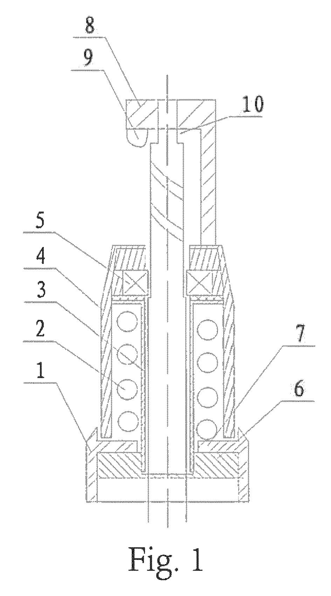

Figure 1 is a structural diagram of an embodiment of the invention.

### Detailed Description of the Preferred Embodiments

The invention is described in detail with the appended drawing and the embodiment.

As shown in FIG.1, a surgical milling cutter bracket includes a retaining base 1 with a through hole. A finger guide 4 with an L-shaped supporting bracket at its top is disposed at the upper part of the retaining base 1. A protrusion 9 is extending downwardly from the end of the short side 8 of the L-shaped supporting bracket, and the lowest point of the protrusion 9 is lower than the lowest point of the cylindrical bit 10 of the milling cutter during the normal operation of the milling cutter. Thus, it is impossible for the object to contact the cylindrical bit, thus avoiding the object be cut unevenly. A T-shaped bolt 3 with a through hole is fixed in the cavity of the finger guide 4. A pressing plate 7 is disposed at the upper part of the retaining base 1, and the bolt 3 passes through the hole of the pressing plate 7 to connect to the nut 6.

A bearing 5 is disposed at the upper part of the finger guide 4. A spring 2 sleeves the bolt 3. The outer surfaces of the retaining base 1 and finger guide 4 are both provided with anti-slip grooves. The bearing 5 provides the milling cutter with an adjacent force point to ensure the smooth motion of the milling cutter. The spring 2 ensures that a certain degree of force is born between the finger guide 4 and the retaining base, thus to avoid misoperation caused by the excessively flexible rotation of the finger guide 4.

When the finger guide 4 is rotated, the bolt 3 and the nut 6 are rotated therewith. Thus, the finger guide 4 drives the L-shaped bracket to rotate to change the motion direction of the milling cutter to facilitate the operation.

The above-mentioned is merely embodiment of the invention and not to be construed in a limiting sense. Without departing from the scope of the invention, various changes, modifications or equal replacements in equivalent embodiments according to the above disclosure will become apparent to those skilled in this art. All simple changes, equal replacements and modifications without departing from the scope of the invention according to the technical substance are seen to fall within the scope of the invention.

## Claims

1. A surgical milling cutter bracket, comprising a retaining base (1) with a through hole, wherein
a finger guide (4) with an L-shaped supporting bracket at a top thereof is disposed at the upper part of the retaining base (1), a protrusion (9) is extending downwardly from the end of the short side (8) of the L-shaped supporting bracket, and the lowest point of the protrusion (9) is lower than the lowest point of a cylindrical bit (10) of the milling cutter during the normal operation of the milling cutter;
the cylindrical bit (10) being provided at the top end of the milling cutter;
a bolt (3) with a center through hole is fixed in a cavity of the finger guide (4); a pressing plate (7) is disposed at the upper part of the retaining base (1), and the bolt (3) passes through the hole of the pressing plate (7) to connect to a nut (6).

2. The surgical milling cutter bracket according to claim 1, **characterized in that** a bearing (5) is disposed at the upper part of the finger guide (4).

3. The surgical milling cutter bracket according to claim 1 or 2, **characterized in that** a spring (2) sleeves the bolt (3).

4. The surgical milling cutter bracket according to claim 3, **characterized in that** the outer surfaces of the retaining base (1) and the finger guide (4) are both provided with anti-slip grooves.

## Patentansprüche

1. Eine Fräserhalteklammer, die einen Haltesockel (1) mit einer Durchgangsbohrung umfasst, wobei eine Fingerführung (4) mit einer L-förmigen Stützklammer darauf auf dem oberen Teil des Haltesockels (1) angeordnet ist, ein Vorsprung (9) sich vom Ende der kurzen Seite (8) der L-förmigen Stützklammer nach unten erstreckt und der niedrigste Punkt des Vorsprungs (9) niedriger ist als der niedrigste Punkt eines zylinderförmigen Einsatzes (10) des Fräsers während des normalen Betriebs des Fräsers; wobei der zylinderförmige Einsatz (10) am oberen Ende des Fräsers bereitgestellt wird; ein Bolzen (3) mit einer Mittendurchgangsbohrung in einem Hohlraum der Fingerführung (4) fixiert ist; eine Druckplatte (7) auf dem oberen Teil des Haltesockels (1) angeordnet ist und der Bolzen (3) durch das Loch der Druckplatte (7) hindurch verläuft, um sich mit einer Mutter (6) zu verbinden.

2. Die Fräserhalteklammer gemäß Anspruch 1, **dadurch gekennzeichnet, dass** ein Lager (5) auf dem oberen Teil der Fingerführung (4) angeordnet ist.

3. Die Fräserhalteklammer gemäß Anspruch 1 oder 2, **dadurch gekennzeichnet, dass** eine Feder (2) den Bolzen (3) bedeckt.

4. Die Fräserhalteklammer gemäß Anspruch 3, **dadurch gekennzeichnet, dass** die Außenflächen des Haltesockels (1) und der Fingerführung (4) beide mit rutschhemmenden Rillen ausgestattet sind.

## Revendications

1. Un support de fraise chirurgicale, comprenant une base de fixation (1) dotée d'un trou débouchant, dans lequel
un guide-doigts (4) doté d'un support profilé en L situé au-dessus du guide-doigts, se trouve sur la partie supérieure de la base de fixation (1), une saillie (9) s'étendant vers le bas depuis l'extrémité du côté court (8) du support profilé en L, et le point le plus bas de la saillie (9) est plus bas que celui d'un foret cylindrique (10) de la fraise lorsqu'elle fonctionne normalement ;
le foret cylindrique (10) étant placé au sommet de la fraise ;
un boulon (3) comprenant un trou débouchant en son centre est fixé dans une cavité du guide-doigts (4) ; une plaque de pression (7) est placée sur la partie supérieure de la base de fixation (1) et le boulon (3) passe dans l'ouverture de la plaque de pression (7) et est relié à un écrou (6).

2. Le support de fraise chirurgicale selon la revendication 1, **caractérisé par** un palier (5) placé sur la partie supérieure du guide-doigts (4).

3. Le support de fraise chirurgicale selon les revendications 1 ou 2; **caractérisé par** un ressort (2) qui entoure le boulon (3).

4. Le support de fraise chirurgicale selon la revendication 3, **caractérisé en ce que** les surfaces extérieures de la base de fixation (1) et du guide-doigts (4) sont composées de rainures antidérapantes.
